# EUROPEAN PATENT SPECIFICATION

(11) **EP 0 385 659 B1**
(45) Date of publication and mention of the grant of the patent: **14.01.1998**
(21) Application number: 90301912.3
(22) Date of filing: 22.02.1990
(51) Int. Cl.: C12N 15/09, C12N 15/31, C12P 21/02, C07K 14/31, C07K 19/00

(54) **Multifunctional polypeptides and method for the preparation thereof**
Multifunktionale Polypeptide und Verfahren zu deren Herstellung
Polypeptides multifonctionnels et leur procédé de préparation

(30) Priority: 03.03.1989 JP 49784/89; 14.11.1989 JP 294033/89
(43) Date of publication of application: 05.09.1990
(60) Divisional application: 97102577.0
(73) Proprietor: FUJITA HEALTH UNIVERSITY, Toyoake-shi, Aichi-ken (JP); TAKARA SHUZO CO. LTD., Fushimi-ku, Kyoto-shi, Kyoto-fu (JP)
(72) Inventor: Sekiguchi, Kiyotoshi, Nagoya-shi, Aichi-ken (JP); Maeda, Toshinaga, Toyoake-shi, Aichi-ken (JP); Titani, Koiti, Kasugai-shi, Aichi-ken (JP); Kimizuka, Fusao, Ohmihachiman-shi, Shiga-ken (JP); Kato, Ikunoshin, Uji-shi, Kyoto-fu (JP)
(74) Representative: Marlow, Nicholas Simon

(56) References cited:
- EP-A- 0 207 751
- EP-A- 0 220 957
- EP-A- 0 291 804
- WO-A-86/00090
- WO-A-89/12677
- NATURE, vol. 309, May 1984, pages 30-33, London, GB; M.D. PIERSCHBACHER et al.: "Cell attachment activity of fibronectin can be duplicated by small synthetic fragments of the molecule"
- PROCEEDINGS OF THE NATIONAL ACADEMY OF SCIENCES, vol. 81, no. 19, October 1984, pages 5985-5988, Washington, US; M.D. PIERSCHBACHER et al.: "Variants of the cell recognition site of fibronectin that retain attachment-promoting activity"
- CELL, vol. 48, March 1987, pages 989-996, New York, US; J. GRAF et al.: "Identification of an Amino Acid Sequence in Laminin Mediating Cell Attachment, Chemotaxis, and Receptor Binding"
- SCIENCE, vol. 238, no. 4826, October 1987, pages 491-497, Washington, US; E. RUOSHLAHTI et al
- CELL, vol. 40, Januar 1985, pages 191-198, New York, US; R. PYTELA et al.: "Identification and isolation of a 140 kd Cell Surface Glycoprotein with Properties Expected of a Fibronectin Receptor"

## Description

### [Industrial field of utilization]

This invention is related to a functional polypeptide, more particularly to a functional polypeptide to which cell adhesive activity has been introduced.

### [Prior art]

With the recent advances in molecular biology, the mechanisms by which cells and the extracellular matrix adhere are coming to be understood on the molecular level. Of the extracellular matrix proteins, Fibronectin (FN) was the first found to contain an essential sequence for cell adhesion. Thus the Arg-Gly-Asp-Ser sequence (RGDS) in the cell-binding domain of FN has been found to be essential for cell adhesion by Ruoslahti et al. (Nature, 309, 30-33, 1984). The RGD part of this sequence is needed for cell adhesion and substitution with other amino acids cannot be done without loss of cell adhesive activity, but the serine can be replaced by, for example, threonine, alanine, cysteine, or valine without loss of activity. However, if substitution is with proline or lysine, the activity is lost. Proteins other than FN which contain the sequence RGD include thrombin, vitronectin, von Willebrand factor, fibrinogen, collagen, discoidin I, λ-phage receptor, and others. It has thus been suggested that the RGD sequence is closely related to protein functions (Ruoslahti et al., Proc. Natl. Acad. Sci. USA, 81, 5985-5988, 1984). However, it is not certain whether the RGD sequence in these molecules confers cell adhesive activity. For example, although fibrinogen has the RGDS sequence, it does not have cell adhesive effects on fibroblasts.

Another example of cell adhesive protein in addition to those named above is laminin. Laminin is a glycoprotein of high molecular weight found in the basement membrane, and it has cell adhesive activity toward a variety of cells in the epithelium. It has been reported (Graf et al., Cell, 48, 989-996, 1987) that the smallest sequence related to cell adhesive is Tyr-Ile-Gly-Ser-Arg (YIGSR). Laminin also has the RGD sequence, but it is not known if the sequence is related to the cell adhesive activity.

In addition, it is known that the Glu-Ile-Leu-Asp-Val (EILDV) sequence in the IIICS domain of FN is related to the adhesion of lymph cells and melanoma cells.

A technique by which a synthetic polypeptide can be bound to a polypeptide originating from FN at the N-terminal of tumor necrosis factor (TNF) has been disclosed (Japanese Laid-Open Patent Application No. 297399/88). The polypeptide bound with TNF is a portion of the sequence of the cell-binding domain of FN, and it contains the sequence RGDS. The synthetic polypeptide obtained as a result of the use of the technique has stronger activity in causing tumor necrosis than TNF. However, in this technique, two functional polypeptides are bound by the use of gene engineering, and the results do not show that the RGDS sequence has the cell adhesive activity within the TNF molecule.

WO-A-8600090 discloses a fused gene encoding (i) a fragment of the diphtheria toxin molecule which confers on the hybrid protein the ability to penetrate the cytoplasmic membrane of a cell and (ii) a sequence encoding a portion of polypeptide ligand conferring selective cell binding activity on the hybrid protein.

Science vol. 238, no. 4826, 1987, pp 491-497 discloses applications of the RGD-adhesion receptor system in the design of peptides and non-peptide systems having improved selectivity and affinity for individual receptors.

Proceedings of the National Academy of Sciences, vol. 81, no. 19, 1984, pp 5985-5988, discloses the role of fibronectin-derived RGDX (where X is an amino acid) sequences in conferring cell adhesive activity on polypeptides.

Briefly, the present invention relates to a synthetic functional polypeptide to the molecule of which has been introduced a peptide which has cell adhesive activity. This invention also relates to a technique based on gene engineering for the introduction of DNA which codes for a peptide which has cell adhesive activity to DNA which codes for another polypeptide, and the use of a plasmid vector which carries said DNA to transform host cells so that they then express the synthetic functional polypeptide.

According to the invention there is provided an artificial polypeptide which prior to modification exhibits 1gG-binding activity and which following intramolecular modification exhibits both the original activity exhibited prior to modification and additionally cell adhesive activity, wherein the polypeptide is *Staphylococcus aureus* Protein A or its fragment modified by insertion of RGD amino acid sequence.

Also according to the invention there is provided genetic material, such as DNA, coding for such a polypeptide.

Also according to the invention there is provided a method for preparing an artificial polypeptide exhibiting IgG-binding activity and cell adhesive activity comprising modifying the DNA sequence of Plasmid pRIT2T by:
cleaving the DNA at the HindIII site corresponding to nucelotide 738 of Protein A; and
inserting a nucleotide sequence coding for the amino acid sequence SLRGDSA at the cleavage site.

Thus, we constructed an expression plasmid by inserting a synthetic DNA which codes for the sequence RGDS into the A region of the DNA sequence which codes for the immunoglobulin G (IgG)-binding region of protein A so that it would match the reading frame and caused this plasmid to be expressed in *Escherichia coli* cells. The IgG-binding activity and the cell adhesive activity of the polypeptide obtained were measured, and it was found that both activities were present. Protein A has the effects of the induction of lymphokines which affect T cells and B cells and the activation of natural killer (NK) cells; it also adsorbs to growth factors of carcinoma cells, etc. By the introduction of the effect of cell adhesive activity to the functions of protein A, these functions can be expected to be further enhanced. Also, because there are RGDS-dependent receptors on the surface of macrophages, which act in defense against infection by phagocytosis (Beppu et al., FEBS Letters, 242, 378-382, 1989), the modified protein A will promote by enhancing the affinity between macrophages and an immunological complex of antigen and IgG.

This invention will be explained in detail as follows.

It is preferable that the sequence to be inserted into a polypeptide freely chosen contains, at the least, the sequence RGD. The amino acid immediately to the C-terminal side of the RGD sequence can be any amino acid other than lysine (K), proline (P), or hydroxyproline (HyP). There seems to be no one-letter code name. There is no particular restriction as to the amino acid immediately to the N-terminal side of the RGD sequence. The insertion site can be any site preferred in the polypeptide, but it is best that the site be one at which such insertion does not cause loss of the original function of the polypeptide. Also, it is preferred that the sequence RGD be inserted in a site at which the tertiary structure can be readily recognized by cell receptors. It is necessary that the gene for the polypeptide into which the RGD sequence is to be inserted is carried by a plasmid which can be expressed when introduced into the host. The DNA sequence corresponding to the RGD sequence is inserted into the coding region of the plasmid so that the reading frame matches, and by the expression of the polypeptide by the host, the desired polypeptide which contains the RGD sequence can be produced. Any of several methods for the insertion of the RGD sequence can be used. For example, if there is a suitable restriction site at the site for insertion, synthetic DNA can be so designed that it can be introduced into that site. Next, after digestion with restriction enzymes, the plasmid DNA can be linked with the synthetic DNA, and then they can be introduced into an appropriate host. Of the recombinants obtained, clones which carry the desired plasmid can be selected. As the selection method, a synthetic DNA probe which is labelled can be used in colony hybridization, or, a synthetic DNA is designed so that when the desired DNA fragment is inserted, a different restriction site can be produced or a previously existing restriction site disappears. In the second two cases, the desired clone can be conveniently selected by investigation of the cleavage pattern of plasmids by restriction enzymes.

When there is no suitable restriction site for the site of insertion, site-directed mutagenesis can be used. Thus, DNA which codes for a polypeptide is inserted into, for example, plasmids pUC118/119, pUC118N/119N, pTV118N/119N, etc., which are expression plasmids which can produce single-stranded DNA.

A primer DNA which has a sequence corresponding to RGD at the center of the sequence, and which is complementary to both sides of the insertion site, is designed and synthesized. With the use of commercial mutation induction kits for DNA. such as the Mutan K kit (Takara Shuzo), recombinants which express the desired polypeptide containing the sequence RGD can be obtained. This method is a general method which can be used to insert the sequence RGD into any position whether or not it is a restriction site.

The invention will be further explained in more detail.

Protein A is a protein which makes up part of the cell wall of Staphylococcus aureus; it contains 473 amino acid residues, and the DNA sequence which codes for it is known. Protein A is produced in precursor form with a signal sequence at its amino terminal. Its mature form has IgG-binding regions named E, D, A, B, and C, in this order, starting from its amino terminal. At its carboxyl terminal, there is the X region, which binds to the cell wall. Plasmid pRIT2T, which expresses the IgG-binding regions D, A, B, and part of C (hereinafter referred to as protein A'), is commercially available from Pharmacia. With the use of this plasmid, it is possible to obtain multifunctional polypeptides like protein A which contain the RGDS sequence by the linking of the DNA sequence which codes for the IgG-binding regions of protein A with the sequence RGDS in an appropriate site so that the DNA reading frames will match. The site which contains the RGDS sequence must be exposed on the surface in a three-dimensional way, and this should be a hydrophilic region. Also, it is necessary that the activity of the protein A not be lost. Because there are a number of binding regions within the IgG-binding region, even if one of the regions is masked, the other regions retain their binding activity. There is a HindIII site (nucleotide 738; Uhl'en et al., J. Biol. Chem., 259, 1695-1702, 1984) in the A region of the gene coding for protein A, and it is convenient to insert the sequence of DNA that codes for RGDS here.

Such a DNA sequence(hereinafter referred to as RODS cassette) can be, for example, of the following structure:

S L R G D S A
5' AGC TTA CGT GGT GAT AGS GCT 3'
3' AT GCA CCA CTA TCG CGA TCG A5'

Here, linkage can be with the HindIII site at the 5' end, and HindIII can be used for cleavage again. At the 3' end, linkage can be with the HindIII site, but the same enzyme cannot be used for cleavage; instead, NheI can be used for cleavage. When this is to be done, the codon which corresponds to alanine, GCT, is added. By doing this, it becomes easy to select the desired plasmid. The upper and lower strands of the RGDS cassette designed as above are synthesized using a DNA synthesizer separately, and the two DNA chains are annealed to form double-stranded DNA. Separately, plasmid pRIT2T, which expresses the IgG-binding region of protein A, can be cleaved by the use of HindIII.

pRIT2T treated in this way and the double-stranded DNA (RGDS cassette) mentioned above can be incubated in the presence of ATP and DNA ligase, giving a reaction mixture containing a plasmid into which the RGDS cassette is inserted so as to match the DNA reading frame. The reaction mixture is used to transform cells of E. coli (such as, for example, strain DH5), and colonies with ampicillin resistance can be selected which, when cultured and extracted, give plasmids. The extract which contains plasmids can be digested with NheI and PstI, the digest can be treated by electrophoresis on agarose, and colonies which have plasmids with the RGDS cassette incorporated in them in the correct orientation can be selected. In the final step, the desired clone can be checked by the sequencing of its DNA bases.

The desired plasmid can be used to transform E. coli N4830-1, and the recombinants obtained can be incubated at 30°C overnight with LB medium or the like which contains ampicillin; the desired product can be expressed by means of a temperature shift. Cells can be harvested from the culture medium, and the cells can be disrupted by the use of ultrasonic waves or the like to give a cell extract. To check for expression, immunoblotting can be used. In this process, total cell protein is separated by sodium dodecyl sulfate (SDS) electrophoresis, and transferred to a nitrocellulose filter; the band of the desired protein can be detected by the use of IgG labelled with an enzyme. The extract is put on a column of agarose bound with IgG (for example, IgG-Sepharose, Pharmacia), and after washing with a buffer of a pH of about 5, the adsorbed fraction is eluted with use of a buffer at the pH of about 3.4. When necessary, further purification is done by the use of highpressure liquid chromatography (HPLC) or fast protein liquid chromatography (FPLC; Pharmacia), to give the desired product.

It is possible to prepare functional polypeptides like protein A which incorporate the sequence RODS in this way, and whether the resulting polypeptide has cell adhesive activity can be checked by, for example, the method of Ruoslahti (Methods in Enzymology, 82, 803-831, 1981). The sample to be tested is dissolved in phosphate-buffered saline (PBS) or the like and allowed to adhere to the wells of a microtitre plate. Then blocking is done with bovine serum albumin (BSA), and either baby hamster kidney (BHK) cells or normal fat kidney (NRK) cells are. placed in the wells and incubated at 37°C. The cells are examined under a microscope for spreading, by which means the cell adhesive activity of the sample to be tested is evaluated. When this was done, protein A' which did not include the sequence RGDS was found not to have cell adhesive activity, but protein A' which did include the sequence RGDS had cell adhesive activity in addition to its IgG-binding activity. Also, protein A' which had the sequence RGES, in which the D of the RGDS sequence had been replaced with E, was prepared in the same way, and protein A' that contained the ROES sequence was found to lack cell adhesive activity. In this way, it was found that cell adhesive activity depended on the presence of the RGDS sequence. When the S of the sequence RGDS was replaced by other amino acids, such as, for example, V, A, T, C, or F, cell adhesive activity was found, so the S of the sequence RODS may be replaced by V, A, T, C, F, or so on. Also, insertion of the RGDS sequence may be in IgG-binding regions other than the A region of protein A, such as in region B, C, or D. When there is no appropriate restriction site, site-directed mutagenesis can be used to insert the desired amino acid sequence in the appropriate position. However, it is difficult to predict if cell adhesive activity will be conferred. It is an important point whether the inserted site has a three-dimensional structure which can be recognized by cell receptors.

### [Examples]

This invention will be explained by reference to examples.

### Example 1.

Construction of a plasmid that expresses protein A' which contains the RGDS sequence:

The DNA chain upstream of the RGDS cassette (5'-AGCTTACGTGGTGATAGCGCT) and the DNA chain downstream (5'-AGCTAGCGCTATCACCACGTA) were each synthesized in a DNA synthesizer (Applied Biosystems, model 380A), and after the removal of protective groups, the DNA was purified by use of HPLC. Then 2 µg of each of these DNA chains was dissolved in 20 µl of TE (10 mM Tris-HCl, pH 8.0, and 1 mM EDTA), and kept for 5 minutes at 70°C before being cooled to room temperature. Separately, 5 µg of a plasmid, pRIT2T (Pharmacia), which expresses protein A' was put into 100 µl of buffer for use with HindIII, and 5 units of HindIII was added. The mixture was incubated for 10 minutes at 30°C to allow digestion. Then the reaction mixture was put on a 1% agarose gel for electrophoresis, and the band at 4.2 kb was purified. Then 1 µg of the RGDS cassette mentioned above was mixed with 25 ng of the plasmid digested with HindIII in 20 µl of a ligation buffer (66 mM Tris-HCl, pH 7.6, 6.6 mM MgCl₂, and 10 mM DTT) containing 0.1 mM ATP and 10% PEG6000. The mixture was incubated with 300 units of DNA ligase at 37°C for 1 hour, and a portion of the reaction mixture was used to transform cells of E. coli DH5. Then the colonies that grew on L-agar plates (1% tryptone, 0.5% yeast extract, 0.5% NaCl, and 1.5% agar) containing 50 µg/ml ampicillan were cultured on L-broth (1% tryptone, 0.5% yeast extract, and 0.5% NaCl), and plasmids were extracted from the cells. The method of Maniatis et al. (Molecular Cloning: A Laboratory Manual, Cold Harbor Laboratory, 1982, p. 368) was used to doubly digest the plasmids with NheI and PstI, the digest was put on 8% polyacrylamide gel for electrophoresis, and the clone which contained plasmids with a band at 400 bp was selected. The plasmid which contained the RGDS cassette in the HindIII site in the A region of the gene for protein A were obtained. Next, these plasmids were doubly digested with the restriction enzymes EcoRI and HindIII, and a plasmid with the RGDS cassette inserted in the correct orientation was selected from the sizes of the fragments obtained. This plasmid was named pAD1, and was used to transform E. coli N4830-1 by the use of the protocol of Pharmacia. The transformants obtained were named E. coli N4830-1/pAD1. Cells of this strain were deposited at the Fermentation Research Institute of the Agency of Industrial Science and Technology as FERM BP-2263.

DNA in which the D of the RGDS sequence was replaced by E (to give the RGES cassette) was chemically synthesized in the same way, and the plasmid in which this DNA was inserted into the same site of plasmid pRIT2T was named pAE1. E. coli N4830-1 into which pAE1 was introduced was named E. coli N4830-1/pAE1.

### Example 2

### Preparation and purification of protein A' containing the RGDS sequence:

Into 5 ml of L-broth, cells of E. coli N4830-1/pAD1 into which pAD1 was introduced were inoculated, and the broth was cultured overnight at 30°C. Then 2.5 ml of the broth was used to inoculate 250 ml of the same medium, and this was cultured at 30°C until the absorbance at 600 nm reached 1, at which time it was added to an equal volume of the same medium heated at 54°C, and culture was shifted to the temperature of 42°C for 90 minutes. This induced the expression of the plasmids. The medium was centrifuged to collect the bacterial cells, which were suspended in 100 ml of TS (50 mM Tris-HCl, pH 7.6, and 150 mM NaCl) and disintegrated ultrasonically. The cell debris was removed by centrifuge and the supernatant was obtained and diluted in five volumes of TS. Then the diluted supernatant was put on a column containing 10 ml of IgG-Sepharose 6FF (Pharmacia). The column was washed with 5 bed volumes of TS and then with 2 bed volumes of 5 mM sodium acetate (pH 5.0) before the substance desired was eluted with the use of 0.5 M acetate (pH 3.4). The eluate was lyophilized, giving 2.5 mg of purified product.

In the same way, 2.3 mg of protein A' containing the RGES sequence was obtained.

### Example 3

### Assay of cell adhesive activity:

The cell adhesive activity of the protein A' which contained the RODS sequence and which contained the RGES sequence obtained in Example 2, of plasma fibronectin, and of protein A' was measured with the use of BHK cells.

### 1. Preparation of samples.

The samples were dissolved in 0.1 M NaHCO₃, and 400 µl of the solution was put into each well of a 24-well flat-bottomed plate (Terumo) and left overnight at 4°C to adsorb. The wells were washed with PBS, and then 500 µl of PBS containing 1% BSA was added to each well. The plate was incubated at room temperature for 3-4 hours, and the wells were washed with PBS before being used in the assay of cell adhesive activity.

### 2. Preparation of cell suspensions.

Subcultures of BHK cells were treated with PBS which contained 0.25% trypsin and 0.02% EDTA at 37°C for 2 minutes to detach the cells from the plate, and the cells were suspended in 10 ml of a 1:1 mixture of ice-cold Dulbecco's modified Eagle's (DME) medium and HEPES physiological saline (137 mM NaCl and 3 mM KCl in 20 mM HEPES buffer, pH 7.1). The suspension was centrifuged and the supernatant was discarded. The cells were suspended in 10 ml of an ice-cold mixture of DME medium and HEPES physiological saline containing 1 mg of soybean trypsin inhibitor (STI). The suspension was centrifuged and the precipitate was washed in an ice-cold mixture of DME and HEPES physiological saline which did not contain STI. These cells were suspended in the same mixture at the concentration of 5 x 10⁵ to 1 x 10⁶/ml.

### 3. Assay of cell adhesive activity.

In each well of the plate to which samples had been caused to adhere, 300 µl of the cell suspension was added, and the plate was incubated at 37°C for 1 hour. Then the wells were washed three times with a mixture of DME medium and HEPES at 37°C, and the non-adhering cells were removed. The remaining cells were fixed with a 4% formalin solution in PBS, and the shapes of the cells were examined by microscopy. The results are shown in Table 1.

**Table 1**

| Sample (5 µg/ml) | Cell adhesive activity | IgG binding activity |
|---|---|---|
| Plasma FN | +++ | - |
| Protein A' (from pRIT2T) | - | + |
| Protein A' with RGDS (from pAD1) | ++ | + |
| Protein A' with RGES (from pAE1) | - | + |

## Claims

1. An artificial polypeptide which prior to modification exhibits lgG-binding activity and which following intramolecular modification exhibits both the original activity exhibited prior to modification and additionally cell adhesive activity, wherein the polypeptide is *Staphylococcus aureus* Protein A or its fragment modified by insertion of the amino acid sequence RGD.

2. An artificial polypeptide according to claim 1 produced by *Escherichia coli* N4830-1/pAD1 (FERM BP-2263).

3. An artificial polypeptide according to claim 2 having an amino acid sequence represented by the following formula:

ADNNFNKEQQNAFYEILNMPNLNEEQRNGFIQSLRGDSASLKDDPSQSANLLSE
AKKLNESQAPK

4. Genetic material, such as DNA, coding for a multifunctional polypeptide according to any preceding claim.

5. A method for preparing an artificial polypeptide exhibiting IgG-binding activity and cell adhesive activity comprising modifying the DNA sequence of Plasmid pRIT2T by:
cleaving the DNA at the HindIII site corresponding to nucelotide 738 of Protein A; and
inserting a nucleotide sequence coding for the amino acid sequence SLRGDSA at the cleavage site.

## Patentansprüche

1. Synthetisches Polypeptid, welches vor der Modifizierung IgG-bindende Aktivität zeigt und welches nach der intramolekularen Modifizierung sowohl die ursprüngliche, vor der Modifizierung gezeigte Aktivität als auch zusätzlich Zellhaftungsaktivität zeigt, wobei das Polypeptid *Staphylococcus aureus* Protein A oder sein durch Einbau der Aminosäuresequenz RGD modifiziertes Fragment ist.

2. Synthetisches Polypeptid nach Anspruch 1, hergestellt durch *Escherichia coli* N4830-1/pAD1 (FERM BP-2263).

3. Synthetisches Polypeptid nach Anspruch 2 mit einer Aminosäuresequenz, dargestellt durch die folgende Formel:

ADNNFNKEQQNAFYEILNMPNLNEEQRNGFIQSLRGDSASLKDDPSQSANLLSE
AKKLNESQAPK

4. Genetisches Material, wie DNA, welches für ein multifunktionelles Polypeptid nach einem vorhergehenden Anspruch kodiert.

5. Verfahren zur Herstellung eines synthetischen Polypeptids, welches IgG-bindende Aktivität und Zellhaftungsaktivität zeigt, welches das Modifizieren der DNA-Sequenz von Plasmid pRIT2T durch:
Spalten der DNA an der HindIII-Stelle, welche zum Nukleotid 738 von Protein A korrespondiert; und
Einbauen einer Nukleotid-Sequenz, welche für die Aminosäuresequenz SLRGDSA kodiert, an der Spaltstelle umfaßt.

## Revendications

1. Polypeptide synthétique qui, avant modification, présente une activité de liaison de IgG et qui, après modification intramoléculaire, présente à la fois l'activité initiale se manifestant avant modification et, en outre, une activité d'adhésion aux cellules, dans lequel le polypeptide consiste en la protéine A de Staphylococcus aureus ou son fragment modififé par insertion de la séquence d'aminoacides RGD.

2. Polypeptide synthétique suivant la revendication 1, produit par Escherichia coli N4830-1/pAD1 (FERM BP-2263).

3. Polypeptide synthétique suivant la revendication 2, ayant une séquence d'aminoacides représentée par la formule suivante :

ADNNFNKEQQNAFYEILNMPNLNEEQRNGFIQSLRGDSASLKDDPSQSANLLSE
AKKLNESQAPK

4. Matériel génétique, tel qu'un ADN, codant pour un polypeptide multifonctionnel suivant l'une quelconque des revendications précédentes.

5. Procédé pour la préparation d'un polypeptide synthétique présentant une activité de liaison de IgG et une activité d'adhésion cellulaire, comprenant la modification de la séquence d'ADN du plasmide pRIT2T par :
clivage de l'ADN au niveau du site HindIII correspondant au nucléotide 738 de la protéine A ; et
insertion d'une séquence de nucléotides codant pour la séquence d'aminoacides SLRGDSA au niveau du site de clivage.
